# EUROPEAN PATENT SPECIFICATION

(11) **EP 1 615 640 B1**
(45) Date of publication and mention of the grant of the patent: **24.01.2007**
(21) Application number: 04750108.5
(22) Date of filing: 14.04.2004
(51) Int. Cl.: A61K 31/436, A61K 38/21, A61P 35/00, A61P 35/02

(54) **ANTINEOPLASTIC COMBINATIONS**
ANTINEOPLASTISCHE ZUSAMMENSETZUNGEN
COMBINAISONS ANTITUMORALES

(30) Priority: 22.04.2003 US 464498 P
(43) Date of publication of application: 18.01.2006
(73) Proprietor: Wyeth, Madison, New Jersey 07940 (US)
(72) Inventor: GIBBONS, James, J., Jr., Westwood, NJ 07675 (US); DUKART, Gary, Ambler, PA 19002 (US); SHERMAN, Matthew, L., Newton, MA 02459 (US)
(74) Representative: Wileman, David Francis
(86) International application number: PCT/US2004/011458
(87) International publication number: WO 2004/093854

(56) References cited:
- WO-A-03/103701
- WO-A-20/04026280
- US-A- 5 362 718
- US-B1- 6 277 983
- BONI J ET AL: "Pharmacokinetics of escalating doses of CCI-779 in combination with 5-fluorouracil and leucovorin in patients with advanced solid tumors" EUROPEAN JOURNAL OF CANCER, PERGAMON PRESS, OXFORD, GB, vol. 37, April 2001 (2001-04), page S68, XP004477483 ISSN: 0959-8049

## Description

This invention relates to the use of combinations of rapamycin 42-ester with 3-hydroxy-2-(hydroxymethyl)-2-methylpropionic acid (CCI-779) or 42-O-(2-hydroxy)ethyl rapamycin and interferon-α, for the preparation of medicaments for use in antineoplastic combination chemotherapy

### BACKGROUND OF THE INVENTION

Rapamycin is a macrocyclic triene antibiotic produced by *Streptomyces hygroscopicus,* which was found to have antifungal activity, particularly against *Candida albicans,* both *in vitro* and *in vivo* [C. Vezina *et al., J. Antibiot.* **28,** 721 (1975); S.N. Sehgal *et al., J. Antibiot.* **28,** 727 (1975); H. A. Baker *et al., J. Antibiot.* **31,** 539 (1978); U.S. Patent 3,929,992; and U.S. Patent 3,993,749]. Additionally, rapamycin alone (U.S. Patent 4,885,171) or in combination with picibanil (U.S. Patent 4,401,653) has been shown to have antitumor activity.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB **3**, 3411 (1989); FASEB **3**, 5256 (1989); R. Y. Calne *et al., Lancet* 1183 (1978); and U.S. Patent 5,100,899]. R. Martel *et al.* [*Can. J. Physiol. Pharmacol. 55,* **48** (1977)] disclosed that rapamycin is effective in the experimental allergic encephalomyelifis model, a model for multiple sclerosis; in the adjuvant arthritis model, a model for rheumatoid arthritis; and effectively inhibited the formation of IgE-like antibodies.

Rapamycin is also useful in preventing or treating systemic lupus erythematosus [U.S. Patent 5,078,999], pulmonary inflammation [U.S. Patent 5,080,899], insulin dependent diabetes mellitus [U.S. Patent 5,321,009], skin disorders, such as psoriasis [U.S. Patent 5,286,730], bowel disorders [U.S. Patent 5,286,731], smooth muscle cell proliferation and intimal thickening following vascular injury [U.S. Patents 5,288,711 and 5,516,781], adult T-cell leukemia/lymphoma [European Patent Application 525,960 A1], ocular inflammation [U.S. Patent 5,387,589], malignant carcinomas [U.S. Patent 5,206,018], cardiac inflammatory disease [U.S. Patent 5,496,832], and anemia [U.S. Patent 5,561,138].

Rapamycin 42-ester with 3-hydroxy-2-(hydroxymethyl)-2-methylpropionic acid (CCI-779) is an ester of rapamycin which has demonstrated significant inhibitory effects on tumor growth in both in vitro and in vivo models. The preparation and use of hydroxyesters of rapamycin, including CCI-779, are disclosed in U.S. Patents 5,362,718 and 6,277,983.

CCI-779 may delay the time to progression of tumors or time to tumor recurrence which is more typical of cytostatic rather than cytotoxic agents. CCI-779 is considered to have a mechanism of action that is similar to that of sirolimus. CCI-779 binds to and forms a complex with the cytoplasmic protein FKBP, which inhibits an enzyme, mTOR (mammalian target of rapamycin, also known as FKBP12-rapamycin associated protein [FRAP]). Inhibition of mTOR's kinase activity inhibits a variety of signal transduction pathways, including cytokine-stimulated cell proliferation, translation of mRNAs for several key proteins that regulate the G1 phase of the cell cycle, and IL-2-induced transcription, leading to inhibition of progression of the cell cycle from G1 to S. The mechanism of action of CCI-779 that results in the G1-S phase block is novel for an anticancer drug.

*In vitro,* CCI-779 has been shown to inhibit the growth of a number of histologically diverse tumor cells. Central nervous system (CNS) cancer, leukemia (T-cell), breast cancer, prostate cancer, and melanoma lines were among the most sensitive to CCI-779. The compound arrested cells in the G1 phase of the cell cycle.

*In vivo* studies in nude mice have demonstrated that CCI-779 has activity against human tumor xenografts of diverse histological types. Gliomas were particularly sensitive to CCI-779 and the compound was active in an orthotopic glioma model in nude mice. Growth factor (platelet-derived)-induced stimulation of a human glioblastoma cell line in vitro was markedly suppressed by CCI-779. The growth of several human pancreatic tumors in nude mice as well as one of two breast cancer lines studied in vivo also was inhibited by CCI-779.

Interferon-alfa is part of a family of naturally occurring proteins, as well as a product produced by recombinant DNA techniques (including interferon alfa-2a and interferon alfa-2b), that has been shown to have antiviral and antitumor properties. While interferon-alfa produces immunomodulatory effects and has antiangiogenic properties, its exact mechanism of action, at least in renal cancer, is unknown. Indications include the treatment of patients with hairy cell leukemia, chronic myelogenous leukemia, follicular lymphoma, cutaneous T cell lymphoma, AIDS-related Kaposi's sarcoma, malignant melanoma, renal cancer, colorectal cancer, other cancers (eg, cervical cancer, ovarian cancer), liver cirrhosis/liver cancer, and the treatment of viral infections, including chronic hepatitis B, chronic hepatitis C, and condylomata acuminata.

### DESCRIPTION OF THE INVENTION

This invention provides the use of CCI-779.and interferon-alfa in preparing a medicament for treating a neoplasm in a mammal in need thereof. In particular, such a combination is useful in the treatment of renal cancer, soft tissue cancer, breast cancer, neuroendocrine tumor of the lung, cervical cancer, uterine cancer, head and neck cancer, glioma, non-small lung cell cancer, prostate cancer, pancreatic cancer, lymphoma, melanoma, small cell lung cancer, ovarian cancer, colon cancer, esophageal cancer, gastric cancer, leukemia, colorectal cancer, Kaposi's sarcoma, liver cancer, and unknown primary cancer.

This invention also provides use of 42-O-(2-hydroxy)ethyl rapamycin and interferon-alfa in preparing a medicament for treating a neoplasm in a mammal in need thereof. The preparation of 42-O-(2-hydroxy)ethyl rapamycin is described in U.S. Patent 5,665,772. The combination of CCI-779 or 42-O-(2-hydroxy)ethyl rapamycin and interferon α may also be used for preparing a medicament for treating an estrogen receptor positive carcinoma in a mammal in need thereof such as an estrogen receptor positive breast or ovarian cancer.

In the use aspect of this invention either the CCI-779 or the interferon α, or both may be provided in subtherapeutically effective amounts. Similarly either the 42-O-(2-hydroxy)ethyl rapamycin or the interferon α or both, may be provided in subtherapeutically effective amounts.

As used in accordance with this invention, the term "treatment" means treating a mammal having a neoplastic disease by providing said mammal an effective amount of a combination of CCI-779 and interferon-alfa with the purpose of inhibiting growth of the neoplasm in such mammal, eradication of the neoplasm, or palliation of the mammal.

As used in accordance with this invention, the term "providing," with respect to providing CCI-779 or 42-O-(2-hydroxy)ethyl rapamycin and interferon-alfa combination (including simultaneous, separate or sequential administration of the components of the combination), means directly administering CCI-779 along with interferon-alfa directly.

The preparation of CCI-779 is described in U.S. Patent 5,362,718. A regiospecific synthesis of CCI-779 is described in US Patent 6,277,983. Interferon-alfa is commercially available as Roferon-A (interferon alfa-2a) and Intron A (interferon alfa-2b).

The combinations of the invention may be in the form of a kit of parts. The invention therefore includes a product containing (a) CCI-779 or 42-O-(2-hydroxy)ethyl rapamycin and (b) interferon (IFN) α as a combined preparation for simultaneous, separate or sequential use in treating a neoplasm in a mammal in need thereof. The invention also includes a pharmaceutical pack containing a course of treatment of a neoplasm for one individual mammal, wherein the pack contains (a) units of CCI-779 or 42-O-(2-hydroxy)ethyl rapamycin in unit dosage form and (b) units of IFNα in unit dosage form.

The results examples illustrate the ability of an illustrative combination of the invention, CCI-779 and interferon alfa, to treat a representative carcinoma, renal cancer. The combination of the invention is useful in treating soft tissue cancer, breast cancer, neuroendocrine tumor of the lung, cervical cancer, uterine cancer, head and neck cancer, glioma, non-small lung cell cancer, prostate cancer, pancreatic cancer, lymphoma, melanoma, small cell lung cancer, ovarian cancer, colon cancer, esophageal cancer, gastric cancer, leukemia, colorectal cancer, Kaposi's sarcoma, liver cancer, and unknown primary cancer.

As typical with chemotherapy, dosage regimens are closely monitored by the treating physician, based on numerous factors including the severity of the disease, response to the disease, any treatment related toxicities, age, and health of the patient. Based on the results obtained with CCI-779, it is projected that initial i.v. infusion dosages will be between about 0.1 and 100 mg/m² when administered on a daily dosage regimen, and between about 1 and 1000 mg/m² when administered on a weekly dosage regimen. Other dosage regimens and variations are foreseeable, and will be determined through physician guidance. It is preferred that CCI-779 is administered by i.v. infusion or orally, preferably in the form of tablets or capsules. Other routes of administration are also feasible, such as via implants, parenterally (besides i.v., such as intraperitoneal and subcutaneous injections), rectally, intranasally, vaginally, and transdermally.

For interferon-alfa, it is projected that initial dosages will be between about 100,000 and 20 million IU daily or between 500,000 and 75 million IU three times weekly. Other dosage regimens and variations are foreseeable, and will be determined through physician guidance. It is preferred that interferon-alfa is administered subcutaneously. Other routes of administration are also feasible, such as intravenously or intramuscularly.

Dosage regimens are expected to vary according to the route of administration. For example, dosages for oral administration are often up to five to tenfold greater than for i.v. administration. The use of concomitant chemotherapeutic agents often allows for dosage reduction of each particular agent, thereby increasing the safety margin of the particular agents.

Oral formulations containing the active compounds of this invention may comprise any conventionally used oral forms, including tablets, capsules, buccal forms, troches, lozenges and oral liquids, suspensions or solutions. Capsules may contain mixtures of the active compound(s) with inert fillers and/or diluents such as the pharmaceutically acceptable starches (e.g. corn, potato or tapioca starch), sugars, artificial sweetening agents, powdered celluloses, such as crystalline and microcrystalline celluloses, flours, gelatins, gums. Useful tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including magnesium stearate, stearic acid, talc, sodium lauryl sulfate, microcrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Preferred surface modifying agents include nonionic and anionic surface modifying agents. Representative examples of surface modifying agents include poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. Oral formulations herein may utilize standard delay or time release formulations to alter the absorption of the active compound(s). The oral formulation may also consist of administering the active ingredient in water or a fruit juice, containing appropriate solubilizers or emulsifiers as needed.

Particularly suitable oral formulations for rapamycin 42-ester with 3-hydroxy-2-(hydroxymethyl)-2-methylpropionic acid are disclosed in USSN 60/411,264 and PCT/US03/29228. Such an oral formulation contains a granulation prepared using a wet granulation process. The granulation contains CCI-779, a water soluble polymer, a pH modifying agent, a surfactant, and an antioxidant. In one embodiment, the formulation contains from 0.1 to 30%, from 0.5 to 25%, from 1 to 20%, from 5 to 15%, or from 7 to 12% (wt/wt) CCI-779, from 0.5 to 50%, from 1 to 40%, from 5 to 35%, from 10 to 25%, or from 15 to 20% (wt/wt) water soluble polymer, from 0.5 to 10%, 1 to 8%, or 3 to 5% (wt/wt) surfactant, and from 0.001% to 1%, 0.01% to 1%, or 0.1% to 0.5% (wt/wt) antioxidant.

The oral formulation may also contain suitable chelating agents, fillers, binders, surfactants, and the like to facilitate the granulation and tableting process. It is preferred that the wet granulation be performed with a hydroalcoholic solvent system comprising water and an alcohol, with ethanol being the preferred alcoholic component.

Typical water soluble polymers include polyvinylpyrrolidone (PVP), hydroxypropylmethylcellulose (HPMC), polyethylene glycol (PEG), and cyclodextrin or mixtures thereof. It is preferred that the water-soluble polymer is PVP, and having a molecular weight of between 2.5 and 60 kilodaltons. Any given oral formulation useful in the invention may contain multiple ingredients of each class of component. For example, an oral formulation containing an antioxidant may contain one or more antioxidants as the antioxidant component.

Acceptable pH modifying agents include citric acid, sodium citrate, dilute HCl, and other mild acids or bases capable of buffering a solution containing CCI-779 to a pH in the range of about 4 to about 6. Acceptable antioxidants include, citric acid, d,l-α-tocopherol, BHA, BHT, monothioglycerol, ascorbic acid, and propyl gallate. It is expected that the antioxidants of the oral formulations used in this invention will be used in concentrations ranging from 0.001% to 3% wt/wt. Chelating agents, and other materials capable of binding metal ions, such as ethylene diamine tetra acetic acid (EDTA) and its salts are capable of enhancing the stability of CCI-779. Surfactants may include polysorbate 80, sodium lauryl sulfate, sodium dodecyl sulfate, salts of bile acids (taurocholate, glycocholate, cholate, deoxycholate) that may be combined with lecithin. Alternatively, ethoxylated vegetable oils, such as Cremophor EL, vitamin E tocopherol propylene glycol succinate (Vitamin E TGPS), polyoxyethylene-polyoxypropylene block copolymers, and poloxamers. Binders, fillers, and disintegrants such as sucrose, lactose, microcrystalline cellulose, croscarmellose sodium, magnesium stearate, gum acacia, cholesterol, tragacanth, stearic acid, gelatin, casein, lecithin (phosphatides), carboxymethylcellulose calcium, carboxymethylcellulose sodium, methylcellulose, hydroxyethylcellulose, hydroxypropylcellulose, hydroxypropylmethycellulose phthalate, noncrystalline cellulose, cetostearyl alcohol, cetyl alcohol, cetyl esters wax, dextrates, dextrin, lactose, dextrose, glyceryl monooleate, glyceryl monostearate, glyceryl palmitostearate, polyoxyethylene alkyl ethers, polyethylene glycols, polyoxyethylene castor oil derivatives, polyoxyethylene stearates, and polyvinyl alcohol may also be incorporated into the oral formulation.

The oral formulation useful in the method of the invention can be prepared by preparing an alcoholic solution comprising CCI-779 and an antioxidant, and an aqueous solution comprising a water-soluble polymer, a surfactant, and a pH modifier, in sufficient quantity to adjust the pH of the aqueous solution to 4 to 6. Suitable alcohols include methanol, ethanol, isopropanol, where ethanol is the preferred alcohol. The solutions were mixed and added to a mixer containing intragranular excipients. Alternatively, the alcoholic and aqueous solutions can be added separately without mixing with each other. Such intragranular excipients comprise binders and fillers to promote dissolution enhancement. Typical intragranular excipients may include microcrystalline cellulose, lactose, and croscarmellose sodium. The solid intragranular excipients are granulated with the solutions in the mixer until a uniform granulation is achieved. The mixer can be a blender with intensifying bar, a low shear granulator or a high shear granulator. The granulation is dried in a fluid bed dryer at approximately 50°C, and milled using a suitable milling device, such as a Fitz mill. The wet granulation and drying can be done in a fluid bed granulator/dryer. The wet granulation can be dried using a tray drying oven. If desired, the dried granulation can be further blended with extragranular fillers and binders, such as microcrystalline cellulose, croscarmellose sodium, and magnesium stearate in a blender, such as a V-blender, before compression into tablets.

Alternatively, some of the water-soluble polymer can be contained in the intragranular excipients, and the aqueous and alcoholic solutions added to the mixer containing the intragranular excipients stepwise. For example, the order of addition to the mixer may be one half of the aqueous solution, followed by the entire alcoholic solution, and then the remainder of the aqueous solution. Other sequences of addition are possible and permissible in these solid oral formulations.

In some cases it may be desirable to administer the compounds directly to the airways in the form of an aerosol.

The compounds may also be administered parenterally or intraperitoneally. Solutions or suspensions of these active compounds as a free base or pharmacologically acceptable salt can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils

Particularly suitable injectable formulations for rapamycin 42-ester with 3-hydroxy-2-(hydroxymethyl)-2-methylpropionic acid are disclosed in US Patent Application No. 10/626,943 and PCT/US03/223276. In this embodiment, the injectable formulation useful in the invention provides a CCI-779 cosolvent concentrate containing an parenterally acceptable solvent and an antioxidant as described above and a parenteral formulation containing CCI-779, composed of CCI-779, an parenterally acceptable cosolvent, an antioxidant, a diluent solvent, and a surfactant. Any given formulation useful in this invention may contain multiple ingredients of each class of component. For example, a parenterally acceptable solvent can include a non-alcoholic solvent, an alcoholic solvent, or mixtures thereof. Examples of suitable non-alcoholic solvents include, *e.g.*, dimethylacetamide, dimethylsulfoxide or acetonitrile, or mixtures thereof. "An alcoholic solvent," may contain one or more alcohols as the alcoholic solvent component of the formulation. Examples of solvents useful in the formulations invention include ethanol, propylene glycol, polyethylene glycol 300, polyethylene glycol 400, polyethylene glycol 600, polyethylene glycol 1000, or mixtures thereof. These cosolvents are particularly desirable because degradation via oxidation and lactone cleavage occurs to a lower extent for these cosolvents. Further, ethanol and propylene glycol can be combined to produce a less flammable product, but larger amounts of ethanol in the mixture generally result in better chemical stability. A concentration of 30 to 100%v/v of ethanol in the mixture is preferred.

In this embodiment, the stability of CCI-779 in parenterally acceptable alcoholic cosolvents is enhanced by addition of an antioxidant to the formulation. Acceptable antioxidants include citric acid, d.l-α-tocopherol, BHA, BHT, monothioglycerol, ascorbic acid, propyl gallate, and mixtures thereof. Generally, the parenteral formulations useful in this embodiment of the invention will contain an antioxidant component(s) in a concentration ranging from 0.001% to 1% w/v, or 0.01% to 0.5% w/v, of the cosolvent concentrate, although lower or higher concentrations may be desired. Of the antioxidants, d,l-α-tocopherol is particularly desirable and is used at a concentration of 0.01 to 0.1% w/v with a preferred concentration of 0.075% w/v of the cosolvent concentrate.

In certain embodiments, the antioxidant component of the formulation of the invention also exhibits chelating activity. Examples of such chelating agents include, e.g., citric acid, acetic acid, and ascorbic acid (which may function as both a classic antioxidant and a chelating agent in the present formulations). Other chelating agents include such materials as are capable of binding metal ions in solution, such as ethylene diamine tetra acetic acid (EDTA), its salts, or amino acids such as glycine are capable of enhancing the stability of CCI-779. In some embodiments, components with chelating activity are included in the formulations of the invention as the sole "antioxidant component". Typically, such metal-binding components, when acting as chelating agents are used in the lower end of the range of concentrations for the antioxidant component provided herein. In one example, citric acid enhanced the stability of CCI-779 when used at a concentration of less than 0.01% w/v. Higher concentrations are less stable solutions and thus, less desirable for products to be subject to long-term storage in liquid form. Additionally, such chelating agents may be used in combination with other antioxidants as part of the antioxidant component of the invention. For example, an acceptable formulation may contain both citric acid and d,l-α-tocopherol. Optimal concentrations for the selected antioxidant(s) can be readily determined by one of skill in the art, based upon the information provided herein.

Advantageously, in certain embodiments of the parenteral formulations useful in the invention, precipitation of CCI-779 upon dilution with aqueous infusion solutions or blood is prevented through the use of a surfactant contained in the diluent solution. The most important component of the diluent is a parenterally acceptable surfactant. One particularly desirable surfactant is polysorbate 20 or polysorbate 80. However, one of skill in the art may readily select other suitable surfactants from among salts of bile acids (taurocholate, glycocholate, cholate, deoxycholate, etc.) which are optionally combined with lecithin. Alternatively, ethoxylated vegetable oils, such as a pegylated castor oil [e.g., such as PEG-35 castor oil which is sold, e.g., under the name Cremophor EL, BASF], vitamin E tocopherol propylene glycol succinate (Vitamin E TGPS), and polyoxyethylene-polyoxypropylene block copolymers can be used in the diluent as a surfactant, as well as other members of the polysorbate family such as polysorbate 20 or 60 Other components of the diluent may include water, ethanol, polyethylene glycol 300, polyethylene 400, polyethylene 600, polyethylene 1000, or blends containing one or more of these polyethylene glycols, propylene glycol and other parenterally acceptable cosolvents or agents to adjust solution osmolarity such as sodium chloride, lactose, mannitol or other parenterally acceptable sugars, polyols and electrolytes. It is expected that the surfactant will comprise 2 to 100% w/v of the diluent solution, 5 to 80% w/v, 10 to 75% w/v, 15 to 60 % w/v, and preferably, at least 5% w/v, or at least 10% w/v, of the diluent solution.

A parenteral formulation useful in the invention can be prepared as a single solution, or preferably can be prepared as a cosolvent concentrate containing CCI-779, an alcoholic solvent, and an antioxidant, which is subsequently combined with a diluent that contains a diluent solvent and suitable surfactant. Prior to use, the cosolvent concentrate is mixed with a diluent comprising a diluent solvent, and a surfactant. When CCI-779 is prepared as a cosolvent concentrate according to this invention, the concentrate can contain concentrations of CCI-779 from 0.05 mg/mL, from 2.5 mg/mL, from 5 mg/mL, from 10 mg/mL or from 25 mg/mL up to approximately 50 mg/ml. The concentrate can be mixed with the diluent up to approximately 1 part concentrate to 1 part diluent, to give parenteral formulations having concentrations of CCI-779 from 1mg/mL, from 5 mg/mL, from 10 mg/mL, from 20 mg/mL, up to approximately 25 mg/ml. For example the concentration of CCI-779 in the parenteral formulation may be from about 2.5 to 10 mg/mL. This invention also covers the use of formulations having lesser concentrations of CCI-779 in the cosolvent concentrate, and formulations in which one part of the concentrate is mixed with greater than 1 part of the diluent, e.g., concentrate: diluent in a ratio of about 1:1.5, 1:2, 1 :3, 1:4 ,1:5, or 1:9 v/v and so on, to CCI-779 parenteral formulations having a CCI-779 concentration down to the lowest levels of detection.

Typically the antioxidant may comprise from about 0.0005 to 0.5% w/v of the formulation. The surfactant may for example comprise from about 0.5% to about 10% w/v of the formulation. The alcoholic solvent may for example comprise from about 10% to about 90% w/v of the formulation.

The parenteral formulations useful in this invention can be used to produce a dosage form that is suitable for administration by either direct injection or by addition to sterile infusion fluids for intravenous infusion.

For the purposes of this disclosure, transdermal administrations are understood to include all administrations across the surface of the body and the inner linings of bodily passages including epithelial and mucosal tissues. Such administrations may be carried out using the present compounds, or pharmaceutically acceptable salts thereof, in lotions, creams, foams, patches, suspensions, solutions, and suppositories (rectal and vaginal).

Transdermal administration may be accomplished through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semi-permeable membrane covering a reservoir containing the active ingredient with or without a carrier, or a matrix containing the active ingredient. Other occlusive devices are known in the literature.

Suppository formulations may be made from traditional materials, including cocoa butter, with or without the addition of waxes to after the suppository's melting point, and glycerin. Water soluble suppository bases, such as polyethylene glycols of various molecular weights, may also be used.

The following examples are illustrative of the present invention.

### Example 1 - CCI-779 and Interferon-α Combination effective against tumor cells

CCI-779 was evaluated in combination with Interferon-alpha (IFN-α) in the HTB-44 human mouse xenograft standard pharmacological test procedure of renal cancer. The human renal cell line HTB-44 (also referred to in the scientific literature as A498) is derived from a patient with a clear cell carcinoma that had lost expression of the von Hippel-Lindau (VHL) gene. These types of tumors are representative of the large majority (≈80%) of sporadic renal cell carcinomas.

CCI-779 was evaluated as a single agent on a weekly schedule against large (>500mg) HTB-44 tumors in nude mouse xenografts. Dosing was from 10 mg/kg to 75 mg/kg intravenously once a week beginning on day 0 when tumors had reached a size of about 500 mm³. All doses tested were similarly effective (30-35 % inhibition of tumor growth) and a dose of 25 mg/kg was chosen to combine with interferon-α.

Similarly, a dose response of IFN-α as a single agent was performed. Doses of 1 million units or 0.5 million units 3 times per week were similarly effective, suggesting that this was the plateau range for maximally effective treatment with IFN-α (data not shown). Therefore, a dose of 1 million units 3 times per week intraperitoneally was chosen to combine with CCI-779 at 25 mg/kg iv once per week. Groups of 10 mice were treated with CCI-779 alone, IFN-α alone, or the combination (Table 1). IFN-α was given on days 1, 3, and 5 and CCI-779 on day 6 of each week for 4 weeks. Dosing began after tumors had reached a size of about 600 mg.

**Table 1. Effect of combination therapy with CCI-779 and Interferon-α on HTB-44 human renal tumor growth in nude mice**

| Tumor mass (mg) | | | | |
|---|---|---|---|---|
| Drug | Week 0 | Week 2 | Week 4 | Week 5 |
| Vehicle control | 585 | 1037 | 1989 | 2255 |
| CCI-779 (25 mg/kg) | 589 (100) | 825 (80) | 1382 (69) | 1539 (68) |
| Interferon α (1 x 10⁶ units) | 586 (100) | 682 (66) | 957 (48) | 1280 (57) |
| CCI-779 + IFN α | 585 (100) | 374 (36) | 401 (20) | 543 (24) |

| | | | | |
|---|---|---|---|---|
| Number in parenthesis = % of vehicle control | | | | |

Treatment with either CCI-779 alone or IFN-α alone resulted in retardation of tumor growth without tumor regression. When combined with IFN-α, CCI-779 induced a 36% regression in the size of HTB-44 renal cell tumors growing in nude mice. As single agents, neither compound induced tumor regression although both showed cytostatic activity. A three fold higher (75mg/kg) dose of CCI-779 than that used in the combination study also did not induce tumor regression. Higher doses of IFN-α are also unlikely to induce regression since there was no difference in single agent IFN at 0.5 or 1.0 million units suggesting that the 1.0 million units used in the combination study was in the plateau range of maximal activity. Taken together these data show that CCl-779 and IFN-α are synergistic in this test procedure in that they were able to achieve an effect (tumor regression) not attainable with single agent treatment.

### Example 2 - CCI-779 and Interferon-α combination active against neoplasm

In xenograft models of RCC such as shown in Example 1, the combination resulted in tumor regression while each agent resulted only in tumor growth inhibition. Thus, the combination of CCI-779 and IFN was evaluated in RCC patients (pts) in a phase 1 study.

In an open-label, ascending-dose, single-arm study, CCI-779 was given IV once weekly, with IFN given subcutaneously 3 times weekly. IFN was given alone the 1 st treatment week. The starting dose levels were 6 million units (MU) IFN and 5 mg CCI-779. CCI-779 dose-escalation steps were 10 mg, 15 mg, and 25 mg. Once the maximum dose of CCI-779 is determined, the dose level of IFN can be escalated to 9 MU (at a reduced CCI-779 dose, if necessary). Dose escalation was based on a safety evaluation of pts (≥6/cohort) after 4 weeks of treatment.

In preliminary results, the number of pts at each dose level was 5 mg: 7 pts, 10 mg: 6, 15 mg: 5, 25 mg: 2. Median age was 55 yrs (range, 40-72 yrs), ECOG performance status 0: 45%, 1: 55%. [ECOG performance status refers to criteria established by the Eastern Cooperative Oncology Group (ECOG) and published, e.g., Oken, M.M., *et al., Toxicity And Response Criteria Of The Eastern Cooperative Oncology Group. Am J Clin Oncol* **5:**649-655, 1982*].* Prior treatment with IL-2: 55%. Of 20 pts, 15 have been on study for 7.6+ mos. CCI-779-related adverse events (AEs) with an overall frequency of ≥20% (n=18) included mucositis (44%), nausea (39%), asthenia (39%), anemia (33%), anorexia (33%), hyperlipidemia (28%), diarrhea (28%), leukopenia (22%), chills (22%), fever (22%), allergic reaction (22%), taste perversion (22%). Gr 3-4 CCI-779-related AEs in ≥2 pts were hyperlipidemia (4), leukopenia (3), hyperglycemia (2). AE-related dose reductions or delays occurred in 7 pts. No drug-related deaths occurred. Partial responses were reported for 2 pts, 5 pts had stable disease, 5 had progressive disease, the remainder were too early to evaluate.

Up to 40 patients are being evaluated at the maximum tolerated dose (MTD). In summary, 71 pts with advanced RCC were enrolled; 27 continue treatment. Patients (73% men, 27% women) had ECOG performance status of 0: 53% and 1: 46% and median age of 59 yrs (range, 35-80); 45% had prior IL-2 treatment. In dose escalation, patients received 6 MU IFNα with CCI-779 at 5 mg (7 pts), 10 mg (6), 15 mg (6), 20 mg (6), or 25 mg (7); 6 pts also received 9 MU IFNα with 15 mg CCI-779. Based on dose-limiting toxicities, 15 mg CCI-779, 6 MU IFNα was selected as the MTD. To date, 33 additional patients have been accrued at the MTD. Grade 3-4 CCI-779-related adverse events with overall frequency ≥ 5% (n=53) were leukopenia (25%), hyperlipidemia (15%), asthenia (13%), AST increase (8%), mucositis (6%), anemia (6%), thrombocytopenia (6%), and rash (6%). Four patients were removed from study due to CCI-779-related toxicity. Approximately 50% of MTD patients have required CCI-779 dose reductions in subsequent cycles. Median time on treatment for all cohorts was 7 mo, 36 have continued for ≥ 6 mo of whom 9 have continued for > 12 mo. Preliminary tumor responses (RECIST) in 55 pts were confirmed partial response, 7 pts (13%); stable disease, 39 (71%, 19 pts ≥ 6 mo); and progressive disease, 9 (16%).

Combination therapy of CCI-779 and IFN has been generally well tolerated in pts with advanced RCC and antitumor activity was observed.

Example 3 - Tablets each containing 2.5 mg of interferon α and also tablets each containing a dose of CCI-779 as mentioned in Example 1 are packaged in a container to provide a course of treatment for a patient.

## Claims

1. Use of CCI-779 and interferon α in preparing a medicament for treating a neoplasm in a mammal in need thereof.

2. Use according to claim 1, wherein the neoplasm is selected from the group consisting of renal cancer, soft tissue sarcoma, breast cancer, neuroendocrine tumor of the lung, cervical cancer, uterine cancer, head and neck cancer, glioma, non-small cell lung cancer, prostate cancer, pancreatic cancer, lymphoma, melanoma, small cell lung cancer, ovarian cancer, colon cancer, esophageal cancer, gastric cancer, leukemia, colorectal cancer, Kaposi's sarcoma, liver cancer and unknown primary cancer.

3. Use according to claim 1 or claim 2, wherein either CCI-779 or the interferon α, or both are provided in subtherapeutically effective amounts.

4. Use of 42-O-(2-hydroxy)ethyl rapamycin and interferon α in preparing a medicament for treating a neoplasm in a mammal in need thereof.

5. Use of CCI-779 and interferon a in preparing a medicament for treating an estrogen receptor positive carcinoma in a mammal in need thereof.

6. Use according to claim 5, wherein the estrogen receptor positive carcinoma is of the breast cancer or ovarian cancer.

7. Use according to claim 5 or claim 6, wherein the CCI-779 or the interferon a, or both are provided in subtherapeutically effective amounts.

8. Use of 42-O-(2-hydroxy)ethyl rapamycin and interferon a in preparing a medicament for treating an estrogen receptor positive carcinoma in a mammal in need thereof.

9. A product containing (a) CCI-779 or 42-O-(2-hydroxy)ethyl rapamycin and (b) interferon α as a combined preparation for simultaneous, separate or sequential use in treating a neoplasm in a mammal in need thereof.

10. Use of CCI-779 or 42-O-(2-hydroxy)ethyl rapamycin in the manufacture of a medicament for treating a neoplasm in a mammal with interferon α.

11. Use of interferon α in the manufacture of a medicament for treating a neoplasm in a mammal with CCI-779 or 42-O-(2-hydroxy)ethyl rapamycin.

12. A pharmaceutical pack containing a course of treatment of a neoplasm for one individual mammal, wherein the pack contains (a) units of CCI-779 and (b) units of interferon α in unit dosage form.

13. A pharmaceutical composition useful in treating a neoplasm in a mammal in need thereof, the composition comprising (a) 42-O-(2-hydroxy)ethyl rapamycin in unit dosage form and (b) units of interferon α in combination or association with a pharmaceutically acceptable carrier.

14. An antineoplastic combination comprising an antineoplastic effective amount of a combination of 42-O-(2-hydroxy)ethyl rapamycin and interferon α.

15. An antineoplastic combination which comprises an antineoplastic effective amount of a combination of CCI-779 and interferon α.

## Patentansprüche

1. Verwendung von CCI-779 und Interferon-α beim Herstellen eines Arzneimittels zur Behandlung eines Neoplasmas in einem Säugetier, das dieser bedarf.

2. Verwendung nach Anspruch 1, wobei das Neoplasma aus der Gruppe bestehend aus Nierenkrebs, Weichteilsarkom, Brustkrebs, neuroendokrinem Tumor der Lunge, Gebärmutterhalskrebs, Gebärmutterkrebs, Kopf- und Halskrebs, Gliom, nicht-kleinzelligem Lungenkarzinom, Prostatakrebs, Bauchspeicheldrüsenkrebs, Lymphom, Melanom, kleinzelligem Lungenkarzinom, Eierstockkrebs, Dickdarmkrebs, Speiseröhrenkrebs, Magenkrebs, Leukämie, kolorektalem Karzinom, Kaposi-Sarkom, Leberkrebs und unbekanntem primärem Karzinom ausgewählt ist.

3. Verwendung nach Anspruch 1 oder 2, wobei entweder CCI-779 oder das Interferon-α oder beide in subtherapeutisch wirksamen Mengen bereitgestellt werden.

4. Verwendung von 42-O-(2-Hydroxy)ethylrapamycin und Interferon-α beim Herstellen eines Arzneimittels zur Behandlung eines Neoplasmas in einem Säugetier, das dieser bedarf.

5. Verwendung von CCI-779 und Interferon-α beim Herstellen eines Arzneimittels zur Behandlung eines östrogenrezeptorpositiven Karzinoms in einem Säugetier, das dieser bedarf.

6. Verwendung nach Anspruch 5, wobei das östrogenrezeptorpositive Karzinom Brustkrebs oder Eierstockkrebs ist.

7. Verwendung nach Anspruch 5 oder 6, wobei das CCI-779 oder das Interferon-α oder beide in subtherapeutisch wirksamen Mengen bereitgestellt werden.

8. Verwendung von 42-O-(2-Hydroxy)ethylrapamycin und Interferon-α beim Herstellen eines Arzneimittels zur Behandlung eines östrogenrezeptorpositiven Karzinoms in einem Säugetier, das dieser bedarf.

9. Produkt, das (a) CCI-779 oder 42-O-(2-Hydroxy)ethylrapamycin und (b) Interferon-α als ein Kombinationspräparat zur gleichzeitigen, separaten oder sequentiellen Verwendung bei der Behandlung eines Neoplasmas in einem Säugetier, das dieser bedarf, enthält.

10. Verwendung von CCI-779 oder 42-O-(2-Hydroxy)ethylrapamycin bei der Herstellung eines Arzneimittels zur Behandlung eines Neoplasmas in einem Säugetier mit Interferon-α.

11. Verwendung von Interferon-α bei der Herstellung eines Arzneimittels zur Behandlung eines Neoplasmas in einem Säugetier mit CCI-779 oder 42-O-(2-Hydroxy)ethylrapamycin.

12. Arzneimittelpackung, die eine Kur zur Behandlung eines Neoplasmas für ein einzelnes Säugetier enthält, wobei die Packung (a) Einheiten von CCI-779 und (b) Einheiten von Interferon-α in Einheitsdosisform enthält.

13. Pharmazeutische Zusammensetzung, die zur Behandlung eines Neoplasmas in einem Säugetier, das dieser bedarf, geeignet ist, wobei die Zusammensetzung (a) 42-O-(2-Hydroxy)ethylrapamycin in Einheitsdosisform und (b) Einheiten von Interferon-α in Kombination oder Verbindung mit einem pharmazeutisch unbedenklichen Trägerstoff umfasst.

14. Antineoplastische Kombination, die eine antineoplastisch wirksame Menge einer Kombination von 42-O-(2-Hydroxy)ethylrapamycin und Interferon-α umfasst.

15. Antineoplastische Kombination, die eine antineoplastisch wirksame Menge einer Kombination von CCI-779 und Interferon-α umfasst.

## Revendications

1. Utilisation de CCI-779 et d'interféron α dans la préparation d'un médicament pour traiter une néoplasie chez un mammifère qui en a besoin.

2. Utilisation selon la revendication 1, dans laquelle la néoplasie est constituée par le cancer du rein, le sarcome des tissus mous, le cancer du sein, la tumeur neuroendocrine du poumon, le cancer cervical, le cancer de l'utérus, le cancer de la tête et du cou, le gliome, le cancer du poumon lorsqu'il n'est pas à petites cellules, le cancer de la prostate, le cancer du pancréas, le lymphome, le mélanome, le cancer du poumon à petites cellules, le cancer des ovaires, le cancer du colon, le cancer de l'oesophage, le cancer de l'estomac, la leucémie, le cancer colorectal, le sarcome de Kaposi, le cancer du foie et le cancer primitif d'origine inconnue.

3. Utilisation slon la revendication 1 ou la revendication 2, dans laquelle soit le CCI-779, soit l'interféron α, soit les deux, sont fournis en des quantités sub-thérapeutiquement efficaces.

4. Utilisation de 42-O-(2-hydroxyéthyl)éthyl rapamycine et d'interféron α dans la préparation d'un médicament pour le traitement d'une néoplasie chez un mammifère qui en a besoin.

5. Utilisation de CCI-779 et d'interféron α dans la préparation d'un médicament pour traiter un carcinome positif aux récepteurs d'oestrogènes chez un mammifère qui en a besoin.

6. Utilisation selon la revendication 5, dans laquelle le carcinome positif aux récepteurs d'oestrogènes est le cancer du sein ou le cancer des ovaires.

7. Utilisation selon la revendication 5 ou la revendication 6, dans laquelle le CCI-779 ou l'interféron α, ou les deux, sont fournis à des quantités sub-thérapeutiquement efficaces.

8. Utilisation de 42-O-(2-hydroxy)éthyl rapacymine et d'interféron α dans la préparation d'un médicament pour traiter un carcinome positif aux récepteurs d'oestrogènes chez un mammifère qui en a besoin.

9. Produit contenant (a) du CCI-779 ou de la 42-O-(2-hydroxy)éthyl rapamycine et (b) de l'interféron α sous forme de préparation combinée pour une utilisation simultanée, séparée ou successive dans le traitement d'une néoplasie chez un mammifère qui en a besoin.

10. Utilisation de CCI-779 ou de 42-O-(2-hydroxy)éthyl rapamycine dans la fabrication d'un médicament pour le traitement d'une néoplasie chez un mammifère avec l'interféron α.

11. Utilisation d'interféron α dans la fabrication d'un médicament pour le traitement d'une néoplasie chez un mammifère avec le CCI-779 ou la 42-O-(2-hydroxy)éthyl rapamycine.

12. Conditionnement pharmaceutique contenant une cure de traitement de néoplasie pour un mammifère individuel, où le conditionnement contient (a) des unités de CCI-779 et (b) des unités d'interféron α sous une forme posologique unitaire.

13. Composition pharmaceutique utile pour traiter une néoplasie chez un mammifère qui en a besoin, la composition comprenant (a) de la 42-O-(2-hydroxy)éthyl rapamycine sous forme posologique unitaire et (b) des unités d'interféron α en combinaison ou association avec un support pharmaceutiquement acceptable.

14. Combinaison antinéoplasique contenant une quantité antinéoplasique efficace d'une combinaison de 42-O-(2-hydroxy)éthyl rapamycine et d'interféron α.

15. Combinaison antinéoplasique qui comprend une quantité antinéoplasique efficace d'une combinaison de CCI-779 et d'interféron α.
